# EUROPÄISCHE PATENTSCHRIFT

(11) **EP 2 416 761 B1**
(45) Veröffentlichungstag und Bekanntmachung des Hinweises auf die Patenterteilung: **11.02.2015**
(21) Anmeldenummer: 10720884.5
(22) Anmeldetag: 10.05.2010
(51) Int. Cl.: A61K 9/14, A61K 31/137, A61P 31/10, A61K 9/26, A61K 9/50, C07C 215/54, C07C 59/255

(54) **DESFESOTERODIN IN FORM EINES WEINSÄURESALZES**
DESFESOTERODIN IN THE FORM OF A TARTARIC ACID SALT
DESFÉSOTÉRODINE SOUS FORME D'UN SEL D'ACIDE TARTRIQUE

(30) Priorität: 11.05.2009 EP 09006357; 20.05.2009 EP 09006848; 16.10.2009 EP 09013104
(43) Veröffentlichungstag der Anmeldung: 15.02.2012
(73) Patentinhaber: ratiopharm GmbH, 89079 Ulm (DE)
(72) Erfinder: FISCHER, Dirk, 89073 Ulm (DE); KOELLNER, Gertraud, 89233 Neu-Ulm (DE); AUER, Gertrud, 89079 Ulm (DE); RIMKUS, Katrin, 80798 München (DE); MUSKULUS, Frank, 88471 Laupheim (DE); BRUECK, Sandra, 85570 Ottenhofen (DE); PAETZ, Jana, 82272 Moorenweis (DE)
(74) Vertreter: Eder, Michael
(86) Internationale Anmeldenummer: PCT/EP2010/002858
(87) Internationale Veröffentlichungsnummer: WO 2010/130392

(56) Entgegenhaltungen:
- WO-A-2007/138440
- DE-A1- 19 932 651
- NILVEBRANT L: "TOLTERODINE AND ITS ACTIVE 5-HYDROXYMETHYL METABOLITE: PURE MUSCARINIC RECEPTOR ANTAGONISTS" PHARMACOLOGY AND TOXICOLOGY, MUNKSGAARD INTERNATIONAL PUBLISHERS, COPENHAGEN, DK, Bd. 90, Nr. 5, 1. Mai 2002 (2002-05-01), Seiten 260-267, XP001188936 ISSN: 0901-9928

## Beschreibung

Die Erfindung betrifft Desfesoterodin in Form eines Weinsäuresalzes, insbesondere in der polymorphen "Form R", sowie ein Verfahren zu dessen Herstellung. In einem zweiten Aspekt betrifft die Erfindung das erfindungsgemäße Desfesoterodin in mikroverkapselter Form.

Fesoterodin ist ein Antimuskarinikum zur Behandlung der überaktiven Blase. Unter Fesoterodin wurden die von den Patienten als sehr belastend empfundenen Symptome der überaktiven Blase deutlich verbessert. In allen klinisch relevanten Endpunkten beider Phase-III-Studien (2, 3) (Dranginkontinenzereignisse/24 h, Miktionshäufigkeit, medianes Miktionsvolumen) ließen sich statistisch signifikante Verbesserungen gegenüber Plazebo erzielen. Fesoterodin wird derzeit unter dem Handelsnamen Toviaz^{®} vermarktet. Fesoterodin ist ein Prodrug. Nach oraler Einnahme erfolgt eine Aktivierung des Prodrugs zum aktiven Metaboliten durch Esterasen im menschlichen Körper.

Der IUPAC-Name von Fesoterodin [INN] ist 2-[(1R)-3-(diisopropylamin)-1-phenylpropyl]-4-(hydroxymethyl)phenyl-isobutyrate. Die chemische Struktur von Fesoterodin wird in nachstehender Formel (1) dargestellt:

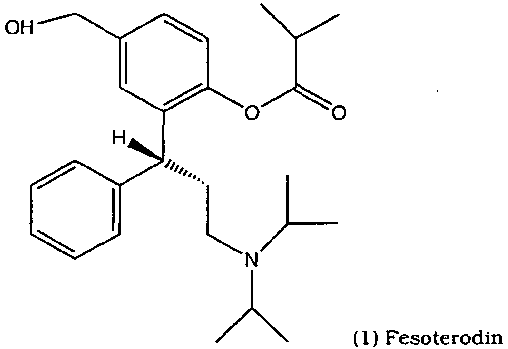

Synthesewege für Fesoterodin sind aus EP 1 077 912 B1 ableitbar. Salze von Fesoterodin sind in EP 1 230 209 B1 beschrieben.

Fesoterodin ist nicht besonders hydrolysestabil. Diesem Umstand Rechnung tragend wurden in WO 2007/141298 Fesoterodin-Tablettenformulierungen vorgeschlagen, die Fesoterodin in Form des Fumarat- oder Hydrogenfumaratsalzes und einen Stabilisator gegen Hydrolyse enthalten, wobei der Stabilisator bevorzugt Xylit ist. Die Verwendung von Xylit in pharmazeutischen Formulierungen ist jedoch häufig unerwünscht. Zudem hat sich gezeigt, dass trotz der Verwendung von Xylit die Verarbeitung von entsprechenden pharmazeutischen Formulierungen aufgrund hygroskopischer Eigenschaften problematisch ist. Ferner sind auch die resultierenden Darreichungsformen im Hinblick auf die Lagerstabilität problematisch, beispielsweise sind zur Verpackung aufwendige Folien mit niedriger Wasserdampfdurchlässigkeit erforderlich.

Aufgabe der vorliegenden Erfindung war es daher, die vorstehenden Nachteile zu überwinden.

Insbesondere war es eine Aufgabe der Erfindung, einen pharmazeutischen Wirkstoff zur Behandlung der überaktiven Blase bereit zu stellen, der keine unerwünschten hygroskopischen Eigenschaften aufweist und vorteilhaft verarbeitet werden kann. Um gute Verarbeitbarkeit zu gewährleisten, sollte der Wirkstoff in einer Form bereit gestellt werden, die gut rieselfähig, gut schüttfähig, nicht hygroskopisch und gut verpressbar ist.

Ferner war es Aufgabe der Erfindung, einen pharmazeutischen Wirkstoff zur Behandlung der überaktiven Blase bereit zu stellen, der im Rahmen einer pharmazeutischen Formulierung eine vorteilhafte Lagerstabilität aufweist. Die vorteilhafte Lagerstabilität soll insbesondere auch dann erreicht werden, wenn zur Verpackung herkömmliche Folien mit einer mittleren bis hohen Wasserdampfdurchlässigkeit verwendet werden.

Zudem war es Aufgabe der Erfindung, einen pharmazeutischen Wirkstoff zur Behandlung der überaktiven Blase bereit zu stellen, der im Wesentlichen eine gleiche Löslichkeit wie Fesoterodinfumarat oder Fesoterodinhydrogenfumarat aufweist und in Folge bei einer oralen Verabreichung im Wesentlichen bioäquivalent zu Fesoterodinfumarat oder Fesoterodinhydrogenfumarat ist.

Die vorstehend genannten Aufgaben konnte überraschend gelöst werden, indem anstelle von Fesoterodinfumarat oder Fesoterodinhydrogenfumarat ein Weinsäuresalz des Fesoterodinmetaboliten "Desfesoterodin" verwendet wird, insbesondere in der nachstehend beschriebenen polymorphen Form R.

Gegenstand der Erfindung ist daher ein Desfesoterodin in Form eines Weinsäuresalzes sowie ein Verfahren zu dessen Herstellung.

Desfesoterodin ist im Stand der Technik bekannt und kann beispielsweise gemäß WO 2005/012227 hergestellt werden. Desfesoterodin ist eine Verbindung gemäß nachfolgender Struktur (2):

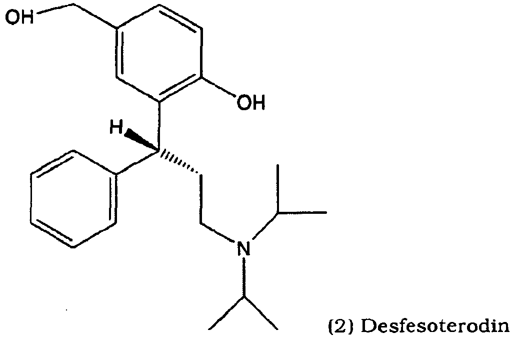

Des Weiteren kann der Ausdruck "Desfesoterodin" auch Enantiomere beziehungsweise Racemate der Verbindung gemäß Struktur (2) umfassen. Der Ausdruck "Desfesoterodin" beschreibt somit 2-(3-Diisopropylamino-1-phenylpropyl)-4-hydroxymethylphenol, insbesondere (R)-2-(3-Diisopropylamino-1-phenylpropyl)-4-hydroxymethylphenol.

Weinsäure ist im Fachgebiet auch als 2,3-Dihydroxybernsteinsäure bekannt. Im Rahmen dieser Erfindung kann Weinsäure als D-(-)-Weinsäure, L-(+)-Weinsäure, meso-Weinsäure oder beliebiges Gemisch davon, z.B. als DL-Racemat, eingesetzt werden.

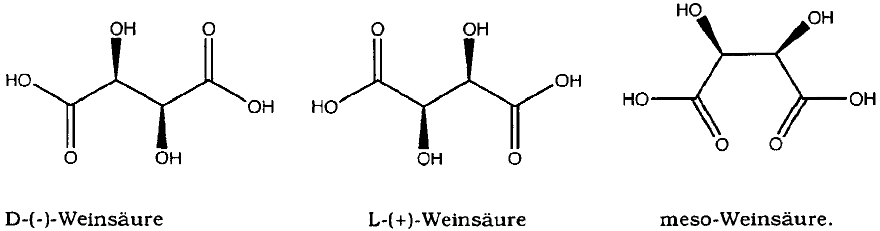

In einer bevorzugten Ausführungsform wird L-(+)-Weinsäure verwendet.

Im erfindungsgemäßen Desfesoterodinsalz beträgt das molare Verhältnis von Desfesoterodin zu Weinsäure etwa 1 : 1.

Grundsätzlich kann das erfindungsgemäße Desfesoterodinsalz beispielsweise in amorpher Form, kristalliner Form oder in Form einer festen Lösung vorliegen. Bevorzugt liegt das erfindungsgemäße Desfesoterodinsalz in kristalliner Form vor.

In einer bevorzugten Ausführungsform wird erfindungsgemäßes kristallines Desfesoterodinsalz in der polymorphen "Form R" verwendet.

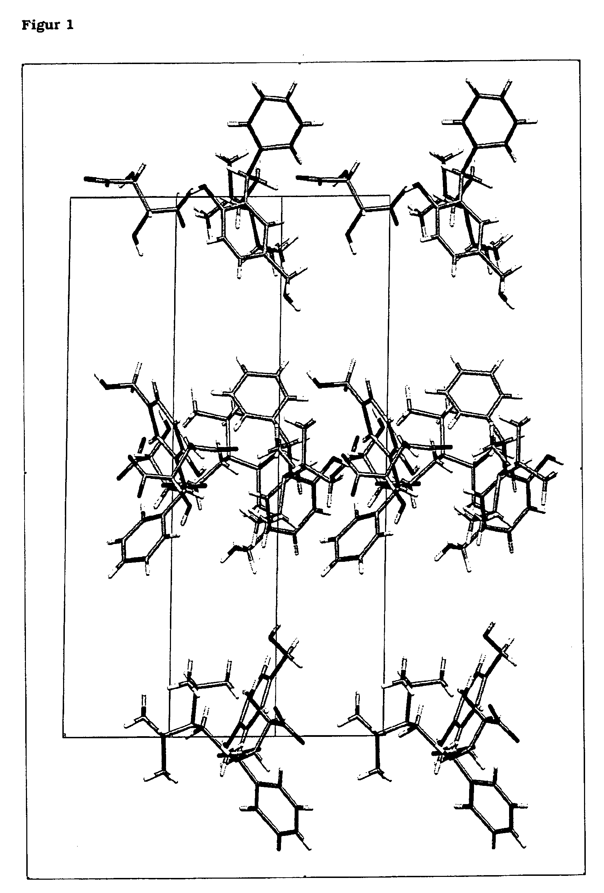

Das erfindungsgemäße kristalline Desfesoterodinsalz, insbesondere die erfindungsgemäße Form R ist hierbei durch ein Kristallgitter mit Schichtstruktur gekennzeichnet. Figur 1 zeigt hierbei die Schichtstruktur in einem Einkristall der Form R. Zwischen den Schichten können sich Lösungsmittelmoleküle einlagern. Es hat sich unerwartet gezeigt, dass insbesondere ein erfindungsgemäßes Desfesoterodinsalz mit Schichtstruktur die vorstehend genannten Aufgaben vorteilhaft löst.

Das erfindungsgemäße kristalline Desfesoterodinsalz, insbesondere die erfindungsgemäße Form R, liegt bevorzugt in einem monoklinen Kristallsystem vor.

Form R ist zudem durch folgende, mittels Einkristallanalyse ermittelte Parameter definiert:

| Raumgruppe: | | *P* 2₁ |
|---|---|---|
| Zellmetrik: | *a* | 15,5 Å |
| | *b* | 11,2 Å |
| | *c* | 22,5 Å |
| | *α* | 90° |
| | *β* | 93,9° |
| | *γ* | 90° |
| Zellvolumen V: | | 3899 Å³ |
| Moleküle pro Elementarzelle Z: | | 4 |

Die erfindungsgemäße "Form R" des Desfesoterodinsalzes ist ferner bevorzugt dadurch gekennzeichnet, dass in den Röntgenbeugungsdiagrammen der Pulver (= Pulverröntgen-diffraktometrie) auf der 2-Theta-Skala mit λ = 1,54 Å (Cu K_{α}) charakteristische Reflexe bei etwa 11,7° +/- 0,2°, 18,4° +/- 0,2° und 18,8° +/- 0,2° auftreten. Weitere charakteristische Reflexe finden sich beispielsweise bei 16,0° +/-0,2°, 16,8° +/- 0,2°, 18,6° +/- 0,2°. 20,6° +/- 0,2°, 20,7° +/- 0,2°, 21,8° +/- 0,2°, 22,0° +/- 0,2°, 23,2° +/- 0,2°, 23,6° +/- 0,2°, 24,9° +/- 0,2° und 29,5° +/- 0,2°.

Die Röntgenbeugungsdiagramme der Pulver werden in Reflexions-Konfiguration (Bragg-Brentano-Geometrie) erhalten. Als Probenträger dienen Träger aus PMMA mit Probenraum von 20,0 mm Durchmesser und 1 mm Tiefe. Die Messungen erfolgen mit einer Röntgenstrahlenquelle mit Kupferanode bei einer Generatorspannung von 40 KV und 40 mA Strom in einem Messkreis von 435,0 mm. Die Detektion erfolgt mit einem schnellen und hochempfindlichen, ortsempfindlichen Detektor (Vantec-1 der Fa. Bruker axs, Karlsruhe).

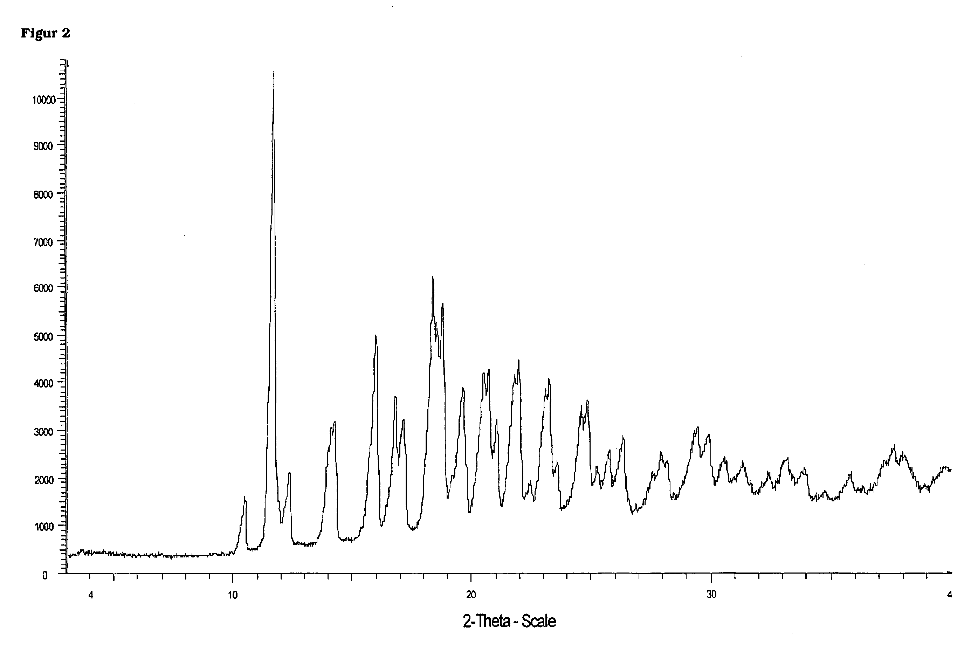

Ein Pulverröntgendiffraktogramm (nachfolgend als "XRPD" bezeichnet) der polymorphen Form R ist in Figur 2 gezeigt. Gegenstand der Erfindung ist somit ein Desfesoterodin-Weinsäuresalz, charakterisiert durch das XRPD gemäß Figur 2.

Das erfindungsgemäße kristalline Desfesoterodinsalz, insbesondere die erfindungsgemäße Form R weist bevorzugt einen Schmelzpunkt von etwa 166 bis 170 °C auf. Die Bestimmung des Schmelzpunktes erfolgt bevorzugt mit dem Gerät Buechi^{®} Melting Point B-545 (mit thermodynamischer Korrektur).

Die erfindungsgemäßen Desfesoterodinsalze liegen (insbesondere im Hinblick auf Verarbeitbarkeit und Bioäquivalenz) bevorzugt in Form einer partikulären Zusammensetzung vor, wobei der mittlere Teilchendurchmesser (D50) üblicherweise 1 bis 500 *µ*m, bevorzugt 5 bis 350 *µ*m, mehr bevorzugt 10 bis 300 *µ*m, besonders bevorzugt 20 bis 250 *µ*m, insbesondere 50 bis 200 *µ*m, beträgt.

Der Ausdruck "mittlerer Teilchendurchmesser" bezieht sich im Rahmen dieser Erfindung - sofern nichts anderes angegeben - auf den D50-Wert des volumenmittleren Teilchendurchmessers, der mittels Laserdiffraktometrie bestimmt wurde. Insbesondere wurde zur Bestimmung ein Mastersizer 2000 von Malvern Instruments verwendet (Nassmessung, 2000 rpm, flüssiges Paraffin als Dispergiermittel, Ultraschall 60 sek., Auswertung gemäß der Fraunhofer Methode). Der mittlere Teilchendurchmesser, der auch als D50-Wert der integralen Volumenverteilung bezeichnet wird, wird im Rahmen dieser Erfindung als der Teilchendurchmesser definiert, bei dem 50 Gew.-% der Teilchen einen kleineren Durchmesser haben als der Durchmesser, der dem D50-Wert entspricht. Ebenso haben dann 50 Gew.-% der Teilchen einen größeren Durchmesser als der D50-Wert.

Gegenstand der vorliegenden Erfindung sind nicht nur die erfindungsgemäßen Weinsäuresalze von Desfesoterodin, sondern auch ein Verfahren zu deren Herstellung. Das erfindungsgemäße Verfahren zur Herstellung der in dieser Anmeldung beschriebenen Weinsäuresalze von Desfesoterodin umfasst die Schritte:
(i) Lösen von Desfesoterodin in einem ersten Lösungsmittel,
(ii) Lösen von Weinsäure in einem zweiten Lösungsmittel,
   wobei das erste und das zweite Lösungsmittel bevorzugt eine Polarität gemessen bei 20 °C von 0,4 bis 1,0 aufweist,
(iii) Vereinigen der Lösungen aus den Schritten (i) und (ii),
(iv) Kristallisieren lassen von Desfesoterodin in Form des Weinsäuresalzes, gegebenenfalls durch Abkühlen der Lösung aus Schritt (iii) auf Temperaturen von -50 °C bis 15 °C.

Grundsätzlich gelten auch für das erfindungsgemäße Verfahren die vorstehend gemachten Erläuterungen zu bevorzugten Ausführungsformen, beispielsweise wird auch im erfindungsgemäßen Verfahren besonders bevorzugt L-(+)-Weinsäure eingesetzt.

Im ersten Verfahrensschritt (i) wird Desfesoterodin (beispielsweise Desfesoterodin gemäß Beispiel 1 von WO 2005/12227 hergestellt) in einem ersten Lösungsmittel gelöst, bevorzugt vollständig gelöst. Der Ausdruck "Lösungsmittel" umfasst hierbei auch Lösungsmittelgemische.

Im zweiten Verfahrensschritt (ii) wird Weinsäure in einem zweiten Lösungsmittel gelöst, bevorzugt vollständig gelöst. Der Ausdruck "Lösungsmittel" umfasst hierbei auch Lösungsmittelgemische.

In Schritt (i) und (ii) kann das gleiche oder auch verschiedene Lösungsmittel verwendet werden. Bevorzugt wird das gleiche Lösungsmittel verwendet. In einer bevorzugten Ausführungsform weist das erste und das zweite Lösungsmittel eine Polarität (gemessen bei 20 °C) von 0,3 bis 0,9, insbesondere von 0,4 bis 0,7 auf.

Beispiele sind für geeignete Lösungsmittel sind Ethylacetat (0,58), Tetrahydrofuran (0,45), Acetonitril (0,65), Isopropanol (0,82), Aceton (0,56) und 2-Butanon (0,51). Besonders bevorzugt ist Tetrahydrofuran (THF) oder 2-Butanon.

In nachstehender Tabelle 1 sind Polaritäten üblicher Lösungsmittel zusammengefasst (eluotrope Reihe).

**Tabelle 1**

| **Substanz** | **Polarität** | **Substanz** | **Polarität** |
|---|---|---|---|
| Fluoralkan | -,25 | Nitropropan | 0,53 |
| n-Hexan | 0,00 | Aceton | 0,56 |
| Petrolether | 0,01 | Dioxan | 0,56 |
| Cyclohexan | 0,04 | Ethylacetat | 0,58 |
| Xylol | 0,26 | Methylacetat | 0,60 |
| Isopropylether | 0,28 | Amylalkohol | 0,61 |
| Isopropylchlorid | 0,29 | Anilin | 0,62 |
| Toluol | 0,29 | DMSO | 0,62 |
| Chlorbenzol | 0,30 | Diethylamin | 0,63 |
| Benzol | 0,32 | Nitromethan | 0,64 |
| Ethylbromid | 0,37 | Acetonitril | 0,65 |
| Diethylether | 0,38 | Pyridin | 0,71 |
| Ethylsulfid | 0,38 | Isopropanol/n-Propanol | 0,82 |
| Chloroform | 0,40 | Ethanol | 0,88 |
| Dichlormethan | 0,42 | Methanol | 0,95 |
| Methylisobutylketon | 0,43 | Ethylenglycol | 1,11 |
| Tetrahydrofuran | 0,45 | | |
| Ethylendichlorid | 0,49 | | |
| Butanon | 0,51 | | |

Im dritten Verfahrensschritt (iii) werden die Lösungen aus Schritt (i) und Schritt (ii) vereinigt. Bevorzugt wird die Lösung aus Schritt (ii) zur Lösung aus Schritt (i) gegeben, insbesondere getropft. In einer alternativen Ausführungsform können die Schritte (i) bis (iii) auch gleichzeitig in einem Reaktionsgefäß erfolgen.

In einer bevorzugten Ausführungsform werden die Schritte (i) bis (iii) bei einer Temperatur des Lösemittels von 20 °C bis 80 °C, bevorzugt von 40 °C bis 75 °C durchgeführt.

Im vierten Verfahrensschritt (iv) erfolgt das Kristallisieren lassen von Desfesoterodin in Form des Weinsäuresalzes bevorzugt in Form R. Die genauen Kristallisationsbedingungen (Temperatur, Zeit) richten sich nach der Wahl des Lösungsmittels. In einer bevorzugten Ausführungsform erfolgt das Kristallisieren lassen durch Abkühlen der Lösung aus Schritt (iii) auf Temperaturen von -80 °C bis 20 °C, mehr bevorzugt von -50 °C bis 15 °C, insbesondere von - 10 °C bis 10 °C.

In einer alternativen Ausführungsform kann das Kristallisieren lassen von Desfesoterodin in Form des Weinsäuresalzes, bevorzugt in Form R, durch Zugabe eines dritten Lösungsmittels (oder Lösungsmittelgemisches) erfolgen. In einer bevorzugten Ausführungsform weist das dritte Lösungsmittel eine Polarität (gemessen bei 20 °C) von 0,0 bis 0,4, insbesondere von 0,01 bis 0,29 auf. Beispiele für geeignete Lösungsmittel sind n-Hexan (0,00), Cyclohexan (0,04), n-Heptan (0,01), Ligroin, Petrolether-Fraktionen (0,01), Methyl-tert.-butylether (0,28) und Isopropylether (0,28).

Im optionalen Verfahrensschritt (v) erfolgt ein Trocknen des resultierenden Desfesoterodins in Form des Weinsäuresalzes bei reduziertem Druck. Die Trocknungsbedingungen (insbesondere Trocknungszeit und Druck) wird bevorzugt so gewählt, dass das resultierende Desfesoterodin in Form des Weinsäuresalzes einen Restlösemittelgehalt von weniger als 3,0 Gew.-% (d.h. z.B. zwischen 0,0001 und 3,0 Gew.-%), mehr bevorzugt von weniger als 2,0 Gew.-%, noch mehr bevorzugt von weniger als 1,0 Gew.-%, besonders bevorzugt von weniger als 0,5 Gew.-%, insbesondere von weniger als 0,1 Gew.-%, aufweist. Üblicherweise wird hierzu ein Druck von 0,1 bis 0,8 bar, bevorzugt von 0.2 bis 0,5 bar verwendet. Die Trocknungszeit beträgt üblicherweise 0,1 bis 10 Stunden, bevorzugt 1 bis 5 Stunden.

Die Trocknung der erfindungsgemäßen Desfesoterodinsalze, insbesondere in Form R, z.B. bis zu dem vorstehend angeführten Restlösemittelgehalt, konnte überraschenderweise ohne Zerstörung des Kristallgitters erfolgen.

Gegenstand der Erfindung sind daher alle vorstehend beschriebenen kristallinen Weinsäuresalze von Desfesoterodin, dadurch gekennzeichnet, dass sie einen Restlösemittelgehalt von weniger als 3,0 Gew.-% (d.h. z.B. zwischen 0,0001 und 3,0 Gew.-%), mehr bevorzugt von weniger als 2,0 Gew.-%, noch mehr bevorzugt von weniger als 1,0 Gew.-%, besonders bevorzugt von weniger als 0,5 Gew.-%, insbesondere von weniger als 0,1 Gew.-% aufweisen. Der Restlösemittelgehalt wird bevorzugt mittels Gaschromatographie, insbesondere mit einem Perkin Elmer System Clarus 500 bestimmt, bevorzugt nach USP 467.

In einer bevorzugten Ausführungsform weist zudem das erfindungsgemäße Desfesoterodinsalz, insbesondere in Form R, einen Wassergehalt von 0,01 bis 4,0 Gew.-%, bevorzugt von 0,02 bis 2,0 Gew.-%, mehr bevorzugt von 0,05 bis 1,5 Gew.-%, noch mehr bevorzugt von 0,10 bis 1,0 % Gew.-%, insbesondere von 0,15 bis 0,9 Gew.-%, auf. Der Restwassergehalt wird nach der Karl Fischer Methode bestimmt, wobei ein Coulometer bei 160 °C verwendet wird. Bevorzugt wird ein Metrohm 831 KF Coulometer mit einer Titrierzelle ohne Diaphragma verwendet. Üblicherweise wird eine Probe von 20 mg Desfesoterodinsalz analysiert. Desfesoterodinsalz mit dem beschriebenen Wassergehalt löst die eingangs beschriebenen Aufgaben überraschend vorteilhaft.

Das erfindungsgemäße Desfesoterodinsalz, insbesondere in Form R, wird üblicherweise zur Herstellung einer pharmazeutischen Formulierung verwendet. Gegenstand der Erfindung ist daher eine pharmazeutische Formulierung, enthaltend erfindungsgemäßes Desfesoterodinsalz, insbesondere in Form R, sowie pharmazeutische Hilfsstoffe. Hierbei handelt es sich um die dem Fachmann bekannten Hilfsstoffe, beispielsweise solche, die im Europäischen Arzneibuch beschrieben sind.

Die Erfinder der vorliegenden Anmeldung haben zudem überraschend festgestellt, dass das erfindungsgemäße Desfesoterodinsalz, insbesondere in Form R, annähernd das gleiche in-vitro Löslichkeitsprofil wie Fesoterodinfumarat zeigt. Im Gegensatz dazu zeigt das aus dem Stand der Technik bekannte Desfesoterodinfumarat ein deutlich schlechteres in-vitro Löslichkeitsprofil.

Gegenstand der Erfindung ist daher die Verwendung von Desfesoterodin in Form eines Weinsäuresalzes zur Herstellung einer pharmazeutischen Formulierung, die bei oraler Einnahme im Wesentlichen bioäquivalent zu einer entsprechenden pharmazeutischen Formulierung mit Fesoterodinfumarat oder Fesoterodinhydrogenfumarat ist.

Grundsätzlich gelten auch für die erfindungsgemäße Verwendung die vorstehend gemachten Erläuterungen zu bevorzugten Ausführungsformen, beispielsweise wird auch bei der erfindungsgemäßen Verwendung besonders bevorzugt L-(+)-Weinsäure eingesetzt. Ebenso wird bevorzugt das erfindungsgemäße Salz in polymorpher Form R eingesetzt.

Der Ausdruck "zu einer entsprechenden pharmazeutischen Formulierung" bedeutet hierbei, dass die erfindungsgemäße Formulierung (enthaltend Desfesoterodin in Form eines Weinsäuresalzes) im Wesentlichen bioäquivalent zu einer Vergleichsformulierung (enthaltend Fesoterodinfumarat oder Fesoterodinhydrogenfumarat) mit im Wesentlichen gleicher Galenik ist.

Der Ausdruck "im Wesentlichen bioäquivalent" bedeutet hierbei, dass die Plasmakonzentrations-Zeit-Profile der beiden Formulierungen bei gleicher Dosis so ähnlich sind, dass hinsichtlich therapeutisch erwünschter oder unerwünschter Wirkungen keine klinisch relevanten Unterschiede zu erwarten sind. Geprüft wird die Bioäquivalenz bevorzugt an Hand der in-vivo Bioverfügbarkeitsparameter AUC, cₘₐₓ und tₘₐₓ. Die Ausdrücke "AUC", "cₘₐₓ' und "tₘₐₓ" sind im Fachgebiet bekannt und werden in Bauer, Frömming, Führer "Lehrbuch der pharmazeutischen Technologie", 8. Auflage, S. 207 - 214 erläutert.

Es ist bevorzugt, dass die Verwendung des erfindungsgemäßen Desfesoterodin in Form eines Weinsäuresalzes beziehungsweise die erfindungsgemäße pharmazeutische Formulierung enthaltend Desfesoterodin in Form eines Weinsäuresalzes, bei oraler Einnahme zu einem AUC-Wert führt, der 90 bis 110 %, mehr bevorzugt 95 bis 105 % des AUC-Werts beträgt, der bei oraler Einnahme einer entsprechenden pharmazeutischen Formulierung mit gleicher Wirkstoffmenge Fesoterodinfumarat oder Fesoterodinhydrogenfumarat erzielt wird. Ebenso wird bevorzugt ein tₘₐₓ-Wert erzielt, der 90 bis 110 %, mehr bevorzugt 95 bis 105 % des tₘₐₓ-Werts beträgt, der bei oraler Einnahme einer entsprechenden pharmazeutischen Formulierung mit gleicher Wirkstoffmenge Fesoterodinfumarat oder Fesoterodinhydrogenfumarat erzielt wird. Ferner wird bevorzugt ein cₘₐₓ-Wert erzielt, der 90 bis 110 %, mehr bevorzugt 95 bis 105 % des cₘₐₓ-Werts beträgt, der bei oraler Einnahme einer entsprechenden pharmazeutischen Formulierung mit gleicher Wirkstoffmenge Fesoterodinfumarat oder Fesoterodinhydrogenfumarat erzielt wird.

Die vorliegende Erfindung ermöglicht folglich die vorstehend erläuterten Nachteile von Fesoterodinfumarat oder Fesoterodinhydrogenfumarat (schwierige Verarbeitbarkeit, problematische Lagerstabilität) im Wesentlichen ohne Änderung der Galenik zu überwinden.

Ferner erfordert der Einsatz von Fesoterodinfumarat oder Fesoterodinhydrogenfumarat - wie vorstehend erläutert - gegebenenfalls ein aufwendiges Packmittel mit einer besonders niedrigen Wasserdampfdurchlässigkeit. Beispielsweise zeigt eine PVC-CTFE Folie (Polyvinylchlorid-Chlorotrifluoroethylen, kommerziell als Aclar^{®} erhältlich) eine besonders niedrige Wasserdampfdurchlässigkeit. Aus technischer Sicht ist es jedoch wünschenswert, dass auch weniger aufwendige Packmittel vorteilhaft verwendet werden können. In Rahmen der vorliegenden Erfindung wurde überraschend gefunden, dass trotz weniger aufwendigeren Packmittel eine pharmazeutische Darreichungsform zur Behandlung der überaktiven Blase mit guter Lagerstabilität bereitgestellt werden konnte. Gegenstand der Erfindung ist somit die Verwendung einer Folie mit einer Wasserdampfdurchlässigkeit von 0,1 g/m²d bis 2,0 g/m²d, bevorzugt mit einer Wasserdampfdurchlässigkeit von 0,2 g/m²d bis 1,0 g/m²d, insbesondere mit einer Wasserdampfdurchlässigkeit von 0,3 g/m²d bis 0,8 g/m²d zur Verpackung einer pharmazeutischen Darreichungsform enthaltend Desfesoterodin, bevorzugt in Form eines Weinsäuresalzes. Bevorzugt handelt es hierbei um eine Darreichungsform von Desfesoterodin zur oralen Verabreichung.

Die Wasserdampfdurchlässigkeit wird im Rahmen dieser Erfindung gemäß Kassis et al.. Pharm. Ind. 43, 1036 (1981) bestimmt werden. Der Parameter "d" bedeutet "Tag".

In einem zweiten Aspekt betrifft die Erfindung eine vorteilhafte Formulierung des erfindungsgemäßen Weinsäuresalzes von Desfesoterodin. Im Stand der Technik wurde zur Formulierung von Fesoterodin üblicherweise sogenannte Matrixtabletten verwendet. Problematisch bei Matrixtabletten ist häufig, dass ein erheblicher Teil des Wirkstoffes (ca. 20 %) in der Regel nicht freigesetzt wird. Eine Aufgabe der vorliegenden Erfindung war es daher, eine Darreichungsform mit modifizierter Freisetzung bereit zu stellen, wobei der Wirkstoff möglichst vollständig freigesetzt werden sollte.

Die im Stand der Technik beschriebenen Herstellverfahren bevorzugen ein klassisches Nassgranulierverfahren. Hier kommt der Wirkstoff üblicherweise für längere Zeit in Kontakt mit Lösungsmitteln. Aufgrund der Empfindlichkeit des Wirkstoffs sollte dies jedoch vermieden werden.

Fesoterodin wird zur Behandlung der überaktiven Blase verwendet. Diese Indikation erfordert es, dass Patienten die Darreichungsformen stets mit sich führen. Die derzeit vermarkteten Toviaz^{®} Tabletten weisen jedoch nur eine Lagerstabilität bis 25 °C auf. Dies ist insbesondere in den Sommermonaten unbefriedigend. Eine weitere Aufgabe der Erfindung war es daher, Fesoterodin in einer Form bereit zu stellen, die für eine Formulierung mit einer Lagerstabilität im praktischen Gebrauch von bis zu 30 °C geeignet ist.

Zudem war es Aufgabe der Erfindung, einen pharmazeutischen Wirkstoff zur Behandlung der überaktiven Blase bereit zu stellen, der im Wesentlichen eine gleiche Löslichkeit wie die in WO 2007/141298 dargestellten Formulierungen, insbesondere die in Tabelle 1 dargestellten Beispielsformulierungen, aufweist und in Folge bei einer oralen Verabreichung im Wesentlichen bioäquivalent dazu ist.

Die vorstehend genannten Aufgaben konnten unerwartet durch Mikroverkapseln von Desfesoterodin in Form des Weinsäuresalzes gelöst werden. Generell gelten in diesem zweiten Aspekt der Erfindung auch alle vorstehend gemachten Erläuterungen zu den bevorzugten Ausführungsformen des Desfesoterodinweinsäuresalzes.

Gegenstand des zweiten Aspekts der Erfindung ist daher ein pharmazeutisches Intermediat, enthaltend mikroverkapseltes Desfesoterodin in Form des Weinsäuresalzes.

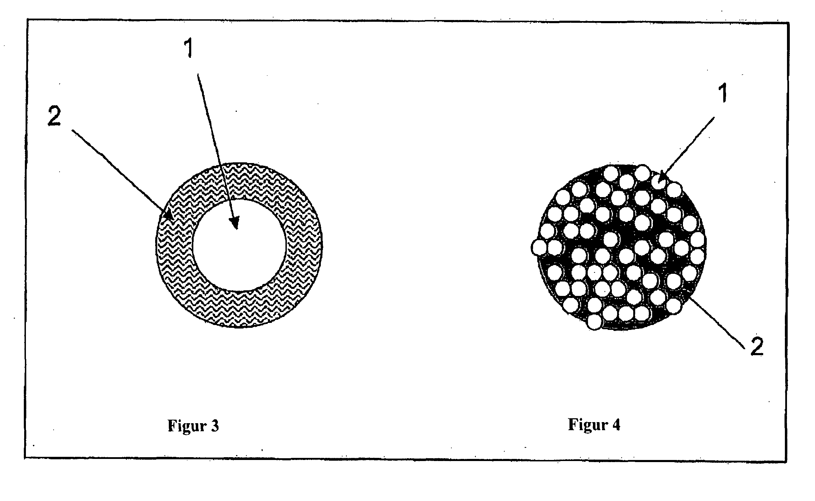

Das erfindungsgemäße mikroverkapselte Desfesoterodinweinsäuresalz wird durch Figur 3 veranschaulicht. In Figur 3 bedeuten:
**1** Wirkstoffpartikel, enthaltend Desfesoterodinweinsäuresalz
**2** Hülle.

Bei dem erfindungsgemäßen pharmazeutischen Intermediat handelt es sich jedoch nicht um sogenannte "Mikrosphärulen". Bei den Mikrosphärulen ist im Gegensatz zu den Mikrokapseln der Wirkstoff in einer Polymermatrix ohne Ausbildung einer Kapselhülle eingebettet. Zum Vergleich werden nicht erfindungsgemäße Mikrosphärulen in Figur 4 abgebildet. In Figur 4 bedeuten:
1 Wirkstoffpartikel, enthaltend Fesoterodin
**2** Matrix.

Somit ist Gegenstand der Erfindung ein pharmazeutisches Intermediat, aufgebaut aus einem Kern (a) und einer Hülle (b),
wobei
(a) der Kern Desfesoterodinweinsäuresalz als Wirkstoff enthält und
(b) die Hülle einen oder mehrere pharmazeutische Hilfsstoffe enthält, welche die Freisetzung des Wirkstoffs modifizieren. Der Kern (a) enthält den Wirkstoff bevorzugt in partikulärer Form, d.h. bei dem Kern handelt es sich bevorzugt um Wirkstoffpartikel, insbesondere um einen oder mehrere Wirkstoffpartikel. Zudem kann der Kern neben Desfesoterodinweinsäuresalz auch pharmazeutische Hilfsstoffe umfassen.

Es ist im Rahmen dieser Erfindung insbesondere bevorzugt, dass der Kern vollständig umhüllt/verkapselt ist. Im Rahmen dieser Erfindung umfasst der Begriff "umhüllt" oder "verkapselt" jedoch auch diejenigen Fälle, wo mindestens 70 %, mehr bevorzugt mindestens 80 %, besonders bevorzugt mindestens 90 %, der Oberfläche des Kerns umhüllt sind.

Der Kern enthält im Allgemeinen Desfesoterodinweinsäuresalz als Wirkstoff. Es ist bevorzugt, dass der Kern im Wesentlichen aus Desfesoterodinweinsäuresalz besteht. Der Ausdruck "im Wesentlichen" weist hier darauf hin, dass der Kern gegebenenfalls noch geringe Mengen an Feuchtigkeit, Lösemittel, pharmazeutische Hilfsstoffe etc. enthalten kann.

Bevorzugt wird für den Kern Desfesoterodinweinsäuresalz mit einem Wassergehalt von 0,1 bis 5 Gew.-%, mehr bevorzugt von 0,3 bis 3 Gew.-%, verwendet.

In einer bevorzugten Ausführungsform enthält der Kern Desfesoterodinweinsäuresalz in granulierter oder komprimierter Form. Das heißt, es wird bevorzugt ein Kern (a) durch Granulation oder Kompression von Desfesoterodinweinsäuresalz gegebenenfalls in Gegenwart von pharmazeutischen Hilfsstoffen, hergestellt werden. Bevorzugt ist eine Kompression, beispielsweise mittels Exzenterpresse. Im Falle der Verwendung von Exzenterpressen wird üblicherweise eine Presskraft von 1 bis 20 kN, bevorzugt von 2,5 bis 10 kN, angewandt.

Im Falle von granulierten oder komprimierten Kerne (a) weisen diese bevorzugt eine gewichtsmittlere Teilchengröße von 0,1 bis 4 mm, mehr bevorzugt von 0,5 bis 3,5 mm, noch mehr bevorzugt von 1,0 bis 3,0 mm, insbesondere von 1,5 bis 2,5 mm auf. Die gewichtsmittlere Teilchengröße wird im Rahmen dieser Anmeldung mittels Siebanalyse bestimmt (bevorzugt unter Verwendung einer Retsch^{®} AS 2000). Es handelt sich hierbei um den D50-Wert.

Im Falle von granulierten oder komprimierten Kerne (a) können diese neben Desfesoterodinweinsäuresalz auch pharmazeutische Hilfsstoffe enthalten. Generell wird diesbezüglich auf die nachstehenden beschriebenen pharmazeutischen Hilfsstoffe verwiesen. Bevorzugt enthalten die Kerne (a) neben Desfesoterodinweinsäuresalz auch Schmiermittel und/oder Zusätze zur Verbesserung der Fließfähigkeit. Besonders bevorzugt enthalten die Kerne (a)
90 bis 100 Gew.-%, insbesondere 92,0 bis 99,0 Gew.-% Desfesoterodinweinsäuresalz;
0 bis 10 Gew.-%, insbesondere 0,5 bis 4 Gew.-% Zusätze zur Verbesserung der Fließfähigkeit;
0 bis 10 Gew.-%, insbesondere 0,5 bis 4 Gew.-% Schmiermittel,
bezogen auf das Gesamtgewicht des Kerns (a).

Die Hülle (b) enthält oder besteht aus einem oder mehreren pharmazeutischen Hilfsstoff(en), welche(r) die Freisetzung des Wirkstoffs modifizieren. Die Hülle (b) liegt hierbei bevorzugt einschichtig vor, d.h. es handelt sich bevorzugt nicht um eine Hülle aus mindestens zwei Schichten.

Unter dem Begriff modifizierte Freisetzung wird im Rahmen dieser Erfindung eine verzögerte Freisetzung (*delayed release),* eine gestaffelte Freisetzung (*repeat action release*), eine hinhaltende Freisetzung *(prolonged release*), eine gleichmäßig hinhaltende Freisetzung (*sustained release*) oder eine lang ausgedehnte Freisetzung *(extended release)* verstanden. Bevorzugt handelt es sich um eine hinhaltende Freisetzung *(prolonged release*)*.*

In einer bevorzugten Ausführungsform umfasst die Hülle (b) die Bestandteile
(b1) einen nicht wasserlöslichen Stoff und
(b2) einen Porenbildner.

Alternativ kann die Hülle (b) im Wesentlichen aus den Bestandteilen (b1) und (b2) bestehen.

Bei der Komponente (b1) handelt es sich bevorzugt um ein nicht-wasserlösliches Polymer oder um einen nicht-wasserlöslichen Stoff mit polymerähnlichen Eigenschaften.

Unter dem Ausdruck "nicht-wasserlöslich" wird im Rahmen dieser Erfindung verstanden, dass der Stoff eine Wasserlöslichkeit von weniger als 10 mg/l, gemessen bei 25 °C, aufweist. Bevorzugt weist der nicht-wasserlösliche Stoff eine Löslichkeit von 8 mg/l oder weniger, insbesondere von 0.01 bis 5 mg/l auf (bestimmt gemäß Säulenelutionsmethode nach EU-Richtlinie RL67-548-EWG, Anhang V Kap. A6).

Das nicht-wasserlösliche Polymer (b1) hat üblicherweise ein gewichtsmittleres Molekulargewicht von 50.000 bis 2.500.000 g/mol, bevorzugt von 150.000 bis 2.000.000 g/mol, mehr bevorzugt von 350.000 bis 1.500.000 g/mol.

Beispiele für geeignete nicht-wasserlösliche Polymere sind Polymere auf Acrylatbasis, z.B. Acrylate, Methacrylate; Cellulosederivate wie Ethylcellulose (EC), Methylcellulose (MC), Celluloseacetylphathalate, Hydroxypropylmethylcellulosephthalat; synthetische Polymere wie Polyvinylalkohol und Derivate davon, Polyvinylacetat, Polyvinylchlorid, Nylon, Polyamid, Polyethylen und Polylactide-co-glycolide. Ebenfalls sind Gemische der genannten Polymere möglich.

Besonders bevorzugt wird Ethylcellulose als nicht-wasserlösliche Polymer (b1) verwendet. Ethylcellulose kann beispielsweise in Form des kommerziell erhältlichen Systems Aquacoat^{®} ECD (FMC BioPolymer, etwa 24,5 bis 29,5 % Ethylcellulose in wässriger Lösung) eingesetzt werden.

Als nicht-wasserlösliche Stoffe (mit polymerähnlichen Eigenschaften) können Wachse und Fette verwendet werden. Geeignete Wachse oder Fette sind bei 25 °C fest. Beispielsweise ist festes Paraffin oder Bienenwachs geeignet. Geeignete Fette sind z.B. Glycerinmonostearat und Glycerinpalmitostearat. Ebenfalls können Gemische davon verwendet werden. Ferner können Gemisch enthaltend nicht-wasserlösliche Polymere und nicht-wasserlösliche Stoffe mit polymerähnlichen Eigenschaften verwendet werden.

Neben dem nicht-wasserlöslichen Stoff (b1) umfasst die Hülle (b) ferner einen Porenbildner (b2). Ein Porenbildner ist im Allgemeinen ein Stoff, der wasserlöslich ist und beim Kontakt der Hülle (b) mit Wasser sich löst, so dass Wasser in die entstandenen Poren eindringen kann. Der Porenbildner weist bevorzugt eine Wasserlöslichkeit von 100 mg/l bei einer Temperatur von 25 °C, besonders bevorzugt von mehr als 250 mg/l auf.

Grundsätzlich sind zwei bevorzugte Ausführungsformen des Porenbildners möglich.

Zum einen kann der Porenbildner ein wasserlösliches Polymer (b2-1) sein. Ferner kann der Porenbildner ein wasserlösliches Salz sein (b2-2).

Geeignete wasserlösliche Polymere weisen bevorzugt hydrophile Gruppen auf. Beispiele für geeignete hydrophile Gruppen sind Hydroxy, Ether, Ester und Amino. Ferner weist das zur Herstellung des Intermediats verwendbare hydrophile Polymer bevorzugt ein gewichtsmittleres Molekulargewicht von 1.000 bis 90.000 g/mol auf, mehr bevorzugt von 2.000 bis 50.000 g/mol auf.

Wird das als Porenbildner verwendete Polymer (b2-1) in Wasser in einer Menge von 2 Gew.-% gelöst, so zeigt die resultierende Lösung bevorzugt eine Viskosität von 0,1 bis 8 mPa/s, mehr bevorzugt von 0,5 bis 7 mPa/s, insbesondere von 1 bis 6 mPa/s, gemessen bei 25 °C.

Das erfindungsgemäße Intermediat kann beispielsweise folgende hydrophile Polymere als Porenbildner umfassen: Polysaccharide, wie Hydroxypropylmethylcellulose (HPMC), Methylcellulose, Hydroxyethylcellulose, Ethylhydroxyethylcellulose, Hydroxypropylcellulose (HPC); Polyvinylpyrrolidon, Polyalkylenglykole, wie Polypropylenglykol oder bevorzugt Polyethylenglykol, Co-blockpolymere des Polyethylenglykols, insbesondere Co-blockpolymere aus Polyethylenglykol und Polypropylenglykol (Pluronic^{®}, BASF) sowie Gemische aus den genannten Polymeren.

Bevorzugt verwendet wird Polyethylenglykol, insbesondere mit einem gewichtsmittleren Molekulargewicht von 2.000 bis 10.000 g/mol.
Alternativ kann der Porenbildner ein wasserlösliches Salz sein (b2-2). Pharmazeutisch verträgliche anorganische Salze sind bevorzugt. Beispiele für geeignete Salze sind NaCl, KCl und Na₂SO₄.

Grundsätzlich sind auch Gemische der genannten Porenbildner möglich.

Die Hülle (b) kann aus den Komponenten (b1) und (b2) bestehen. In einer bevorzugten Ausführungsform enthält die Hülle neben dem nicht-wasserlöslichen Stoff (b1) und dem Porenbildner (b2) noch zusätzlich ein Polymer mit einer pH-abhängigen Wasserlöslichkeit (b3) und/oder Weichmacher (b4).

Es ist bevorzugt, dass die Komponente (b3) ein Polymer ist, das im Sauren eine schlechtere Wasserlöslichkeit als im Neutralen oder Alkalischen aufweist. Die Polymere (b3) weisen üblicherweise ein zahlenmittleres Molekulargewicht von > 10.000 bis 90.000, bevorzugt von 20.000 bis 70.000 g/mol auf. Beispiele für geeignete Polymere mit pH-abhängiger Wasserlöslichkeit sind Celluloseacetattrimellitat (CAT), Polvyinylacetatphthalat, Hydroxypropylmethylcellulosephthalat (HPMCP), insbesondere mit einem gewichtsmittleren Molekulargewicht von 40.000 bis 60.000. Carboxymethylethylcellulose (CMEC), Polyvinylacetatphthalat (PVAP), anionische Methacrylate (z.B. Eudragit^{®} L30), Celluloseacetatphthalat (CAP) und Schellack.

Es sind grundsätzlich auch Polymere denkbar, die sowohl die Definition von (b2) als auch von (b3) erfüllen. Im Rahmen dieser Erfindung wird jedoch ein Polymer nur entweder als Komponente (b2) oder als Komponente (b3) verwendet.

Wie vorstehend beschrieben enthält in einer bevorzugten Ausführungsform die Hülle ferner als Komponente (b4) Weichmacher. Unter "Weichmacher" sind im allgemeinen Stoffe zu verstehen, welche in der Lage sind, die Glasübergangstemperatur des nicht-wasserlöslichen Polymers (b1) zu senken (d.h. ein Gemisch aus (b1) und (b4) weist eine niedrige Glasübergangstemperatur auf als Komponente (b1) alleine).

Beispiele für geeignete Weichmacher sind Glycerin, Citrate wie Triethylcitrat, Tributylcitrat, Acetylcitrat, Phthalate, wie Dibutylphthalat, Diethylphthalat, Dimethylphthalat, Sebacate wie Dibutylsebacat oder Diethylsebacat. Ebenfalls können Alkylenglykole wie Ethylenglykol, Propylenglykol, Butylenglykol (1,4-Butandiol) oder Polyalkylenglykole wie Polyethylenglykol, bevorzugt mit einem gewichtsmittleren Molekulargewicht von 300 bis 1500 g/mol, verwendet werden. Bevorzugt werden Triethylcitrat und/oder Dibutylsebacat verwendet, insbesondere wird Triethylcitrat als Weichmacher (b4) verwendet. Ebenfalls können Gemische der genannten Weichmacher verwendet werden.

Die Hülle (b) enthält bevorzugt 70 bis 99 Gew.-%, mehr bevorzugt 75 bis 95 Gew.-%, besonders bevorzugt 80 bis 93 Gew.-%, insbesondere 85 bis 92 Gew.-% nicht-wasserlösliches Polymer (b1).

Die Hülle (b) enthält weiterhin bevorzugt 1 bis 20 Gew.-%, mehr bevorzugt 2 bis 15 Gew.-%. besonders bevorzugt 3 bis 12 Gew.-% Porenbildner (b2).

Die Hülle (b) enthält zudem bevorzugt 0 bis 20 Gew.-% Polymer mit einer pH-abhängigen Löslichkeit (b3).

Die Hülle (b) enthält zudem bevorzugt 0 bis 20 Gew.-%, mehr bevorzugt 2 bis 15 Gew.-%, besonders bevorzugt 3 bis 12 Gew.-% einen Weichmacher (b4).

Die genannten Einzelbereiche können beliebig kombiniert werden. Beispielsweise enthält die Hülle (b)
(b1) 75 bis 95 Gew.-%, mehr bevorzugt 80 bis 90 Gew.-% nicht-wasserlösliches Polymer
(b2) 0,1 bis 20 Gew.-% Porenbildner, mehr bevorzugt 0,5 bis 15 Gew.-%, insbesondere 0,1 bis 10 Gew.-% und
(b3) 0 bis 20 Gew.-% Polymer mit einer pH-abhängigen Löslichkeit und/oder
(b4) 0 bis 20 Gew.-%, mehr bevorzugt 2 bis 15 Gew.-%, insbesondere 3 bis 12 Gew.-% Weichmacher,
bezogen auf das Gesamtgewicht der Hülle (b).

Es ist ferner bevorzugt, dass die Hülle (b) im Wesentlichen aus den Bestandteilen (b1), (b2) und (b4) besteht.

Die eingesetzten Mengen von Kern (a) und Hülle (b) werden bevorzugt so gewählt, dass der Kern vollständig umhüllt ist. Der Kern ist somit bevorzugt vollständig eingekapselt.

Die erfindungsgemäßen Intermediate liegen bevorzugt in Form einer partikulären Zusammensetzung vor, wobei die gewichtsmittlere Teilchengröße 0.15 bis 5 mm, 0,6 bis 4,0 mm, noch mehr bevorzugt von 1,5 bis 3,3 mm, insbesondere von 1,8 bis 2,8 mm auf. Die gewichtsmittlere Teilchengröße wird wie vorstehend beschrieben mittels Siebanalyse bestimmt. Die zweite Ausführungsform findet bevorzugt Anwendung, wenn die Kerne (a) wie vorstehend granuliert oder komprimiert wurden.

Üblicherweise beträgt im erfindungsgemäßen Intermediat das Gewichtsverhältnis von Kern (a) zu Hülle (b) 15 : 1 bis 1 : 5, bevorzugt 10 : 1 bis 1 : 3, mehr bevorzugt 8 : 1 bis 1 : 2, insbesondere 6 : 1 bis 1: 1. Die zweite Ausführungsform findet bevorzugt Anwendung, wenn die Kerne (a) wie vorstehend granuliert oder komprimiert wurden.

Das erfindungsgemäße Intermediat (d.h. das mikroverkapselte Desfesoterodinweinsäuresalz) ist im Allgemeinen durch ein Verfahren herstellbar, wobei die Polymerhülle auf den Desfesoterodinweinsäuresalz-Kern aufgebracht wird.

Gegenstand der Erfindung ist daher ein Verfahren zur Herstellung des erfindungsgemäßen pharmazeutischen Intermediats umfassend die Schritte:
(i) Bereitstellen von Desfesoterodinweinsäuresalz in partikulärer Form;
(ii) Bereitstellen einer Lösung, die hüllenbildende pharmazeutische Hilfsstoffe enthält;
(iii) Aufsprühen der Lösung aus Schritt (ii) auf die Teilchen von Desfesoterodinweinsäuresalz; und
(iv) Entfernung des Lösungsmittels.

Es ist bevorzugt, dass im Schritt (i) in des erfindungsgemäßen Verfahrens Desfesoterodinweinsäuresalz mit einer gewichtsmittleren Teilchengröße von 0,1 bis 4 mm, mehr bevorzugt von 0,5 bis 3,5 mm, noch mehr bevorzugt von 1,0 bis 3,0 mm, insbesondere von 1,5 bis 2,5 mm eingesetzt werden.

Im Schritt (ii) werden pharmazeutische Hilfsstoffe, die zur Bildung der Hülle (b) geeignet sind, in einem Lösungsmittel oder Lösungsmittelgemisch gelöst oder suspendiert, bevorzugt vollständig gelöst. Insbesondere handelt es sich bei diesen pharmazeutischen Hilfsstoffen um die vorstehend erläuterten Komponenten (b1), (b2), (b3) und/oder (b4). Vorstehende Ausführungen zu dem erfindungsgemäßen Intermediat finden auch auf das erfindungsgemäße Verfahren Anwendung.

Als Lösungsmittel eignen sich z.B. Wasser, Alkohol (z.B. Methanol, Ethanol, Isopropanol), Dimethylsulfoxid (DMSO), Aceton, Butanol, Ethylacetat, Heptan, Pentanol oder Gemische daraus. Bevorzugt wird ein Ethanol/Wasser Gemisch oder insbesondere Wasser verwendet. Die hüllenbildenden Stoffe, bevorzugt die Komponenten (b1), (b2) und gegebenenfalls (b3) und/oder (b4), liegen üblicherweise in der Lösung/Suspension in einer Konzentration von 5 % bis 95 Gew.-%, vorzugsweise 60 % bis 90 Gew.-%, bezogen auf das Gesamtgewicht der Lösung vor.

Im anschließenden Schritt (iii) erfolgt ein Aufsprühen der Lösung aus Schritt (ii) auf die Desfesoterodinweinsäuresalz-Partikel. Bevorzugt erfolgt das Aufsprühen im Wirbelschichtbett.

Im Schritt (iv) wird das Lösungsmittel entfernt, bevorzugt vollständig entfernt. Die Entfernung des Lösemittels erfolgt bevorzugt durch hohe Temperatur und/oder niedrigen Druck. Der Restlösemittelgehalt in der Hülle (b) beträgt bevorzugt kleiner 2 Gew.-%.

Es ist bevorzugt, dass die Schritte (i) bis (iv) in einem Arbeitsgang und bevorzugt in einem Gerät erfolgen. Die Verweilzeit von Lösungsmittel auf dem Wirkstoff soll möglichst kurz, bevorzugt weniger als 10 Minuten, insbesondere weniger als 5 Minuten, sein.

In einer bevorzugten Ausführungsform wird das erfindungsgemäße Verfahren in einem Wirbelschichtgranulator durchgeführt, beispielsweise in einem Glatt^{®} GPCG 3 (Glatt GmbH, Deutschland).

Neben dem vorstehend beschriebenen Verfahren sind auch Intermediate, erhältlich nach dem erfindungsgemäßen Verfahren, Gegenstand dieser Erfindung.

Das erfindungsgemäße Intermediat (d.h. das erfindungsgemäße eingekapselte Desfesoterodinweinsäuresalz) wird üblicherweise zur Herstellung einer pharmazeutischen Formulierung verwendet.

Gegenstand der Erfindung ist daher eine pharmazeutische Formulierung, enthaltend erfindungsgemäßes Intermediat sowie pharmazeutische Hilfsstoffe.

Hierbei handelt es sich um die dem Fachmann bekannten Hilfsstoffe, beispielsweise solche, die im Europäischen Arzneibuch beschrieben sind.

Das Verhältnis Wirkstoff zu Hilfsstoffe wird bevorzugt so gewählt, dass die resultierende Formulierungen
0,1 bis 50 Gew.-%, mehr bevorzugt 0,5 bis 40 Gew.-%, noch mehr bevorzugt 1 bis 30 Gew.-%, insbesondere 2 bis 20 Gew.-% Desfesoterodinweinsäuresalz, und 50 bis 99,9 Gew.-%, 60 bis 99,5 Gew.-%, mehr bevorzugt 70 bis 99 Gew.-%, insbesondere 80 bis 98 Gew.-% pharmazeutisch verträgliche Hilfsstoffe enthalten.

Bei diesen Angaben wird die Menge an Hüllmaterial (b) und gegebenenfalls auch die Menge an Hilfsstoffe im Kern (a), die zur Herstellung des erfindungsgemäßen Intermediats verwendet wurde, als Hilfsstoff gerechnet. Das heißt, die Menge an Wirkstoff bezieht sich auf die Menge an Desfesoterodinweinsäuresalz, die in der Formulierung enthalten ist/sind.

Beispiele für verwendete Hilfsstoffe sind Sprengmittel, Trennmittel, Pseudo-Emulgatoren, Füllstoffe, Zusätze zur Verbesserung der Pulverfließfähigkeit, Gleitmittel, Netzmittel und/oder Schmiermittel.

Die erfindungsgemäße Formulierung kann Füllstoffe enthalten. Unter Füllstoffe sind im Allgemeinen Stoffe zu verstehen, die zur Bildung des Tablettenkörpers bei Tabletten mit geringen Wirkstoffmengen dienen. Das heißt, Füllstoffe erzeugen durch "Strecken" der Wirkstoffe eine ausreichende Tablettiermasse. Füllstoffe dienen üblicherweise also dazu, eine geeignete Tablettengröße zu erhalten.

Beispiele für bevorzugte Füllstoffe sind Lactose, Lactosederivate, Stärke, Stärkederivate, behandelte Stärke, Talkum, Calciumphosphat, Saccharose, Calciumcarbonat, Magnesiumcarbonat, Magnesiumoxid, Calciumsulfat, hydrogeniertes Pflanzenöl und Kaolin. Ebenfalls kann silifizierte mikrokristalline Cellulose (z.B. Prosolv^{®}, Rettenmaier & Söhne, Deutschland) verwendet werden. Die bevorzugt verwendete silifizierte mikrokristalline Cellulose ist kommerziell erhältlich weist einen Siliciumdioxidgehalt von 1 bis 3 Gew.-%, bevorzugt von 2 Gew.-% auf. Ebenfalls können Kombinationen der genannten Füllstoffe verwendet werden, beispielsweise wird eine Kombination aus Lactose und mikrokristalliner Cellulose vorteilhaft verwendet.

Füllstoffe werden üblicherweise in einer Menge von 1 bis 90 Gew.-%, mehr bevorzugt von 10 bis 80 Gew.-%, mehr bevorzugt von 20 bis 60 Gew.-%, bezogen auf das Gesamtgewicht der Formulierung, verwendet.

Als Sprengmittel werden im Allgemeinen Stoffe bezeichnet, die den Zerfall einer Darreichungsform, insbesondere einer Tablette, nach Einbringen in Wasser beschleunigen. Geeignete Sprengmittel sind z.B. organische Sprengmittel wie Carrageenan, Croscarmellose und Crospovidon. Alternativ verwendet werden alkalische Sprengmittel. Unter alkalischen Sprengmitteln sind Sprengmittel zu verstehen, die beim Lösen in Wasser einen pH-Wert von mehr als 7,0 erzeugen, beispielsweise NaHCO₃ oder Na₂CO₃.

Sprengmittel werden üblicherweise in einer Menge von 0 bis 20 Gew.-%, mehr bevorzugt 1 bis 15 Gew.-%, insbesondere 2 bis 10 Gew.-% verwendet, bezogen auf das Gesamtgewicht der Formulierung. Sofern granulierte oder komprimierte Kerne (a) verwendet werden ist bevorzugt, dass kein Sprengmittel verwendet wird.

Ein Beispiel für einen Zusatz zur Verbesserung der Pulverfließfähigkeit ist disperses Siliciumdioxid, z.B. bekannt unter dem Handelsnamen Aerosil^{®}. Bevorzugt wird Siliciumdioxid mit einer spezifischen Oberfläche von 50 bis 400 m²/g, bestimmt nach Gasadsorption gemäß Ph. Eur., 6. Auflage 2.9.26., verwendet, insbesondere falls granulierte oder komprimierte Kerne (a) verwendet werden.

Zusätze zur Verbesserung der Pulverfließfähigkeit werden üblicherweise in einer Menge von 0,1 bis 3 Gew.-%, bezogen auf das Gesamtgewicht der Formulierung, verwendet.

Ferner können Schmiermittel verwendet werden. Schmiermittel dienen im Allgemeinen zur Verringerung der Gleitreibung. Insbesondere soll die Gleitreibung vermindert werden, die beim Tablettieren einerseits zwischen den sich in der Matrizenbohrung auf und ab bewegenden Stempeln und der Matrizenwand sowie andererseits zwischen Tablettensteg und Matrizenwand besteht. Geeignete Schmiermittel stellen z.B. Stearinsäure, Adipinsäure, Natriumstearylfumarat (Pruv^{®}) und/oder Magnesiumstearat dar.

Schmiermittel werden üblicherweise in einer Menge von 0,1 bis 3 Gew.-%, bezogen auf das Gesamtgewicht der Formulierung, verwendet.

Weiterhin können Trennmittel verwendet werden. Unter Trennmitteln werden üblicherweise Stoffe verstanden, welche die Agglomeration im Kernbett vermindern. Beispiele sind Talkum, Silicagel, und/oder Glycerolmonostearat. Trennmittel werden üblicherweise in einer Menge von 0 bis 3 Gew.-%, bezogen auf das Gesamtgewicht der Formulierung, verwendet.

Es liegt in der Natur von pharmazeutischen Hilfsstoffen, dass diese teilweise mehrere Funktionen in einer pharmazeutischen Formulierung wahrnehmen. Im Rahmen dieser Erfindung gilt zur unzweideutigen Abgrenzung daher bevorzugt die Fiktion, dass ein Stoff, der als ein bestimmter Hilfsstoff verwendet wird, nicht zeitgleich auch als weiterer pharmazeutischer Hilfsstoff eingesetzt wird. Beispielsweise wird mikrokristalline Cellulose - sofern als Füllstoff eingesetzt - nicht auch zusätzlich als Sprengmittel eingesetzt (obwohl mikrokristalline Cellulose auch eine gewisse Sprengwirkung zeigt).

Es ist ein Vorteil der vorliegenden Erfindung, dass auf Feuchtestabilisatoren verzichtet werden kann. Die erfindungsgemäße Formulierung enthält bevorzugt keine Feuchthaltemittel, ausgewählt aus Glucose, Glucosederivaten und Zuckeralkoholen. Die erfindungsgemäße Formulierung enthält besonders bevorzugt kein Isomalt, Xylitol, Sorbitol, Polydextrose, Dextrose und Gemische daraus.

Die erfindungsgemäße Formulierung kann in unterschiedlichen Darreichungsformen verabreicht werden. Bevorzugt wird sie zu Tabletten verpresst. Alternativ kann die erfindungsgemäße Formulierung in Kapseln, Sachets oder Stickpacks abgefüllt werden.

Bevorzugt findet die erfindungsgemäße pharmazeutische Formulierung in Form von Tabletten Verwendung. Gegenstand der Erfindung ist daher ein Verfahren zur Herstellung einer Tablette, enthaltend die erfindungsgemäße pharmazeutische Formulierung, umfassend die Schritte
(a) Vermischen des erfindungsgemäßen Intermediats mit pharmazeutischen Hilfsstoffen;
(b) Kompression zu Tabletten, gegebenenfalls unter Zusatz weiterer pharmazeutischer Hilfsstoffe; und
(c) gegebenenfalls Befilmung der Tabletten.

Im Schritt (a) werden erfindungsgemäßes Intermediat und weitere (vorstehend beschriebene) pharmazeutische Hilfsstoffe vermischt. Die Vermischung kann in üblichen Mischern erfolgen. Beispielsweise kann die Vermischung in Zwangsmischern oder Freifallmischern erfolgen (z.B. mittels Turbula^{®} T10B (Bachofen AG, Schweiz)). Die Mischzeit kann beispielsweise 1 bis 15 Minuten betragen.

In Schritt (b) erfolgt eine Kompression zu Tabletten. Die Kompression kann mit im Stand der Technik bekannten Tablettiermaschinen erfolgen. Die Kompression erfolgt bevorzugt in Abwesenheit von Lösungsmitteln.

Beispiele für geeignete Tablettiermaschinen sind Exzenterpressen oder Rundlaufpressen. Beispielsweise kann eine Fette 102i (Fette GmbH, Deutschland) verwendet werden. Im Falle von Rundlaufpressen wird üblicherweise eine Presskraft von 2 bis 40 kN, bevorzugt von 2,5 bis 35 kN angewandt. Im Falle von Exzenterpressen wird üblicherweise eine Presskraft von 1 bis 20 kN, bevorzugt von 2,5 bis 10 kN, angewandt. Beispielsweise wird die Korsch^{®} EKO verwendet.

Im optionalen Schritt (c) des erfindungsgemäßen Verfahrens werden die Tabletten aus Schritt (b) befilmt. Hierbei können die im Stand der Technik üblichen Verfahren zur Befilmung von Tabletten Anwendung finden.

Für die Befilmung werden bevorzugt makromolekulare Stoffe verwendet, beispielsweise modifizierte Cellulosen, Polymethacrylate, Polyvinylpyrrolidon, Polyvinylacetatphthalat, Zein und/oder Schellack.

Die Schichtdicke des Überzugs beträgt bevorzugt 10 bis 100 *µ*m.
Es ist bevorzugt, dass der gegebenenfalls aufgebrachte Film im Wesentlichen keine Auswirkungen auf die Freisetzung hat. Somit handelt es sich bevorzugt um Filme ohne Einfluss auf die Wirkstofffreisetzung. Im Rahmen dieser Erfindung werden bevorzugt weder magensaftresistente Filmüberzüge noch Retardüberzüge verwendet.

Die Tablettierbedingungen werden im erfindungsgemäßen Verfahrens ferner bevorzugt so gewählt, dass die resultierenden Tabletten ein Verhältnis von Tablettenhöhe zu Gewicht von 0,005 bis 0,3 mm/mg, besonders bevorzugt 0,05 bis 0,2 mm/mg aufweisen.

Ferner weisen die resultierenden Tabletten bevorzugt eine Härte von 50 bis 250 N, besonders bevorzugt von 80 bis 200 N, insbesondere von 110 bis 170 N auf. Die Härte wird gemäß Ph.Eur. 6.0, Abschnitt 2.9.8, bestimmt.

Zudem zeigen die resultierenden Tabletten bevorzugt eine Friabilität von kleiner 5 %, besonders bevorzugt von kleiner 3 %, insbesondere kleiner 2 %, auf. Die Friabilität wird gemäß Ph.Eur. 6.0, Abschnitt 2.9.7. bestimmt.

Schließlich weisen die erfindungsgemäßen Tabletten üblicherweise eine Gleichförmigkeit des Gehalts *(Content Uniformity)* von 90 bis 110 %, bevorzugt von 95 bis 105 %, insbesondere von 98 bis 102 % vom durchschnittlichen Gehalt auf. Die "Content Uniformity" wird gemäß Ph. Eur.6.0, Abschnitt 2.9.6. bestimmt.

Der zweite Aspekt der Erfindung soll nochmals durch folgende Punkte zusammengefasst werden:
1. Pharmazeutisches Intermediat, enthaltend mikroverkapseltes Desfesoterodinweinsäuresalz. Hierbei wird auf alle vorstehend gemachten Erläuterungen zu bevorzugten Ausführungsformen (z.B. kristalline Form R) des erfindungsgemäßen Desfesoterodinweinsäuresalzes Bezug genommen.
2. Pharmazeutisches Intermediat, aufgebaut aus einem Kern (a) und einer Hülle (b), wobei
   (a) der Kern Desfesoterodinweinsäuresalz als Wirkstoff enthält und
   (b) die Hülle einen oder mehrere pharmazeutische Hilfsstoffe enthält, welche die Freisetzung des Wirkstoffs modifizieren,
   wobei bevorzugt das Gewichtsverhältnis von Kern (a) zu Hülle (b) 15 : 1 bis 1 : 5 beträgt.
3. Intermediat gemäß Punkt 2, wobei die Hülle die Bestandteile
   (b1) nicht wasserlöslichen Stoff, bevorzugt nicht-wasserlösliches Polymer, und
   (b2) Porenbildner umfasst.
4. Intermediat nach Punkt 3, wobei die Hülle zusätzlich die Bestandteile
   (b3) Polymer mit einer pH-abhängigen Löslichkeit und/oder
   (b4) Weichmacher umfasst.
5. Intermediat nach Punkt 3 oder 4, wobei die Hülle
   (b1) 75 bis 95 Gew.-% nicht-wasserlösliches Polymer
   (b2) 0,1 bis 20 Gew.-% Porenbildner
   und
   (b4) 0 bis 20 Gew.-% Weichmacher umfasst.
6. Intermediate nach einem der Punktel bis 5, wobei die Intermediate in Form einer partikulären Zusammensetzung vorliegen und der gewichtsmittlere Teilchendurchmesser 0,5 bis 5,0 mm beträgt.
7. Intermediat nach einem der Punkte 1 bis 6. wobei es sich bei dem Wirkstoff um Desfesoterodinweinsäuresalz in vorstehend beschriebener polymorpher Form R handelt.
8. Verfahren zur Herstellung eines pharmazeutischen Intermediats gemäß einem der Punktel bis 7 umfassend die Schritte:
   (i) Bereitstellen von Desfesoterodinweinsäuresalz in partikulärer Form;
   (ii) Bereitstellen einer Lösung, die hüllenbildende pharmazeutische Hilfsstoffe enthält;
   (iii) Aufsprühen der Lösung aus Schritt (ii) auf die Teilchen von Desfesoterodinweinsäuresalz; und
   (iv) Entfernung des Lösungsmittels.
9. Verfahren nach Punkt 8, wobei im Schritt (i) Desfesoterodinweinsäuresalz mit einer mittleren Teilchengröße von 0,5 bis 5,0 mm eingesetzt werden.
10. Intermediat, erhältlich nach einem Verfahren gemäß Punkt 8 oder 9.
11. Pharmazeutische Formulierung, enthaltend ein Intermediat nach einem der Punktel bis 7 und 10 sowie einen oder mehrere pharmazeutische Hilfsstoffe.
12. Pharmazeutische Formulierung gemäß Punkt 11, wobei der Wirkstoffgehalt mehr als 5,0 Gew.-% bis 15 Gew.-%, bezogen auf das Gesamtgewicht der Formulierung, beträgt.
13. Verfahren zur Herstellung einer Tablette enthaltend eine pharmazeutische Formulierung gemäß Punkt 11 oder 12, umfassend die Schritte:
   (a) Vermischen eines Intermediats nach einem der Punktel bis 7 und 10 mit pharmazeutischen Hilfsstoffen:
   (b) Kompression zu Tabletten, gegebenenfalls unter Zusatz weiterer pharmazeutischer Hilfsstoffe; und
   (c) gegebenenfalls Befilmung der Tabletten.
14. Tablette mit einer Friabilität von kleiner 3 %, mit einer Gleichförmigkeit des Gehalts von 95 bis 105 % und mit einer Härte von 80 bis 200 N, enthaltend eine pharmazeutische Formulierung gemäß Punkt 11 oder 12.
15. Mikroverkapseltes Desfesoterodinweinsäuresalz zur Behandlung der überaktiven Blase.

Die Erfindung soll durch die nachfolgenden Beispiele veranschaulicht werden.

### BEISPIELE

### Beispiel 1: Desfesoterodin-Tartratsalz aus THF

In einem 500 ml Kolben wurden 3,6 g (10,5 *µ*mol) Desfesoterodin (als freie Base, hergestellt nach WO2005/012227) in 120,0 ml THF gelöst und auf 55 °C erwärmt. Zu der Lösung wurde über 2h 1,5 g (10,0 *µ*mol) L-(+)-Weinsäure, gelöst in 60,0 ml THF, zugetropft. Nach Zugabe von ∼60% L-(+)-Weinsäure begann das Tartratsalz zu kristallisieren. Nach Beendigung der Zugabe von L-(+)-Weinsäure wurde die Suspension auf Raumtemperatur gekühlt und anschließend in der Kälte (5 °C) über Nacht gelagert. Die erhaltenen Kristalle wurden abgesaugt, mit kaltem THF gewaschen und bei 50 °C / 400 mbar für 24 Stunden getrocknet. Es wurden 3,75 g (76,2%) eines weißen kristallinen Feststoffes erhalten.

### Beispiel 2: Desfesoterodin-Tartratsalz aus 2-Butanon

In einem 250 ml Kolben wurden 0,6 g (1,8 *µ*mol) Desfesoterodin (als freie Base, hergestellt nach WO2005/012227) in 20,0 ml THF gelöst und auf 75 °C erwärmt. Zu der Lösung wurde über 2h 0,25 g (1,7 *µ*mol) L-(+)-Weinsäure, gelöst in 10,0 ml 2-Butanon, zugetropft. Nach Zugabe von ∼30% L-(+)-Weinsäure begann das Tartratsalz auszufallen. Nach Beendigung der Zugabe von L-(+)-Weinsäure wurde die Suspension langsam auf Raumtemperatur gekühlt. Die erhaltenen Kristalle wurden abgesaugt, mit kaltem 2-Butanon gewaschen und bei 50 °C / 400 mbar für 24 Stunden getrocknet. Es wurden 0,65 g (79,6%) eines weißen kristallinen Pulvers erhalten.

### Beispiel 3: Umkristallisieren aus THF für Einkristallanalyse

1 g des Desfesoterodin-Tartratsalzes gemäß Beispiel 1 wurde in 125 ml THF suspendiert und auf 55 °C erwärmt bis eine klare Lösung erhalten wurde. Diese Lösung wurde langsam bis auf 0 °C gekühlt und die erhaltenen Kristalle wurden abgesaugt. (Ausbeute 680 mg).

### Vergleichsbeispiel 1: Desfesoterodin-Fumaratsalz aus THF

In einem 250 ml Kolben wurden 0,19g (1,6 *µ*mol) Fumarsäure in 20 ml THF bei 55°C gelöst. Zu der Lösung wurden über 2h 0,62g (1,8 *µ*mol) Desfesoterodin (als freie Base, hergestellt nach WO2005/012227) gelöst in 10 ml THF getropft. Nach Beendigung der Zugabe des Desfesoterodins wurde die Suspension langsam auf Raumtemperatur gekühlt. Die erhaltenen Kristalle wurden abgesaugt, mit kaltem THF gewaschen und bei 50 °C / 400 mbar für 24 Stunden getrocknet. Es wurden 0,68 g (91,0%) eines weißen kristallinen Pulvers erhalten.

### Beispiel 4: in-vitro Löslichkeit und Lagerstabilität

Das in-vitro Löslichkeitsverhalten von Fesoterodinfumarat (kristallin, kommerziell erhältlich), Desfesoterodinfumarat gemäß Vergleichsbeispiel 1 und Desfesoterodintartrat gemäß erfindungsgemäßen Beispiel 1 wurde untersucht:

| pH | Fesoterodinfumarat | Desfesoterodinfumarat | Desfesoterodintartrat |
|---|---|---|---|
| 4,5 | gut | schlecht | gut |
| 10 | gut | schlecht | gut |

| | | | |
|---|---|---|---|
| Einteilung Löslichkeitsverhalten: schlecht: < 20 mg/ml mittel: 20 bis 60 mg/ml gut: > 60 bis 80 mg/ml sehr gut: > 80 mg/ml | | | |

Ferner wurde die Lagerstabilität von Desfesoterodintartrat in einem Stress-Test bestimmt. Es zeigte sich, dass Desfesoterodin sowohl in fester Form als auch in wässriger Lösung bei pH 4,5 sowie pH 10 eine vorteilhafte Lagerstabilität aufwies.

### Beispiel 5: Mikroverkapseltes Desfesoterodinweinsäuresalz

### Kern (a):

| | | |
|---|---|---|
| Desfesoterodinweinsäuresalz | 4,00 | 96,85% |
| Aerosil^{®} | 0,08 | 1,94% |
| Magnesiumstearat | 0,05 | 1,21% |

Für einen Ansatz von 300 g Kernen, wurde Desfesoterodinweinsäuresalz zusammen mit Aerosil^{®} und Magnesiumstearat 10 Minuten auf einem Freifallmischer (Turbula^{®} T10B) gemischt und anschließend auf einer Exzenterpresse zu Kernen verpresst (Korsch^{®} EKO).

Die Kerne wiesen dabei ein Gehalt von 4 mg Wirkstoff auf und besaßen einen gewichtsmittleren Durchmesser von ungefähr 2 mm.

300 g dieser Kerne (a) wurden mit 145 g eines Hülle-bildenden Films (b) aus Aquacoat^{®} ECD 77% (b1), Triethylcitrat 6% (b4), Polyethylenglykol 2% (b2) und 15% Wasser in einer Wirbelschichtanlage (Glatt GPC 3.1) überzogen.

Anschließend wurden jeweils ein Intermediat (umhüllter Kern) in Microcellac^{®} (75% Lactose, 25% mikrokristalline Cellulose) und 1% Magnesiumstearat eingeschlossen und verpresst, um Tabletten mit einem Gesamtgewicht von 320 mg und einer Härte von 150 N zu erhalten (Form 12,5 x 6,5 mm, Gehalt 4mg).

## Patentansprüche

1. (R)-2-(3-Diisopropylamino-1-phenylpropyl)-4-hydroxymethylphenol (Desfesoterodin) in Form eines Weinsäuresalzes, wobei das molare Verhältnis von Desfesoterodin zu Weinsäure etwa 1 : 1 beträgt.

2. Desfesoterodinsalz gemäß Anspruch 1, wobei es sich um L-(+)-Weinsäure handelt.

3. Desfesoterodinsalz gemäß Anspruch 1 oder 2 in kristalliner Form.

4. Desfesoterodinsalz gemäß Anspruch 3, wobei das Kristallgitter eine Schichtstruktur aufweist.

5. Desfesoterodinsalz gemäß Anspruch 3 oder 4, wobei das Kristallsystem monoklin ist.

6. Desfesoterodinsalz gemäß einem der Ansprüche 3 bis 5, wobei das Desfesoterodinsalz im Röntgenpulverdiffraktogramm auf der 2-Theta-Skala λ = 1,54 Å (Cu K_{α}) charakteristische Reflexe bei 11,7° +/- 0,2°, 18,4° +/- 0,2° und 18,8° +/- 0,2° aufweist (polymorphe "Form R").

7. Desfesoterodinsalz gemäß einem der Ansprüche 3 bis 6, wobei der Schmelzpunkt im Bereich von 166 °C bis 170 °C liegt.

8. Verfahren zur Herstellung von (R)-2-(3-Diisopropylamino-1-phenylpropyl)-4-hydroxymethylphenol (Desfesoterodin) in Form eines Weinsäuresalzes gemäß einem der Ansprüche 1 bis 7, umfassend die Schritte
(i) Lösen von Desfesoterodin in einem ersten Lösungsmittel,
(ii) Lösen von Weinsäure in einem zweiten Lösungsmittel,
wobei das erste und das zweite Lösungsmittel bevorzugt eine Polarität gemessen bei 20 °C von 0,4 bis 0,9 aufweist,
(iii) Vereinigen der Lösungen aus den Schritten (i) und (ii),
(iv) Kristallisierenlassen von Desfesoterodin in Form des Weinsäuresalzes, gegebenenfalls durch Abkühlen der Lösung aus Schritt (iii) auf Temperaturen von -50 °C bis 15 °C;
wobei die Schritte (i) bis (iii) gegebenenfalls bei einer Temperatur von 20 °C bis 80 °C durchgeführt wurden.

9. Verfahren gemäß Anspruch 8, wobei das erste und zweite Lösungsmittel identisch sind und aus Butanon oder Tetrahydrofuran ausgewählt wurden.

10. Verfahren gemäß einem der Ansprüche 8 oder 9, umfassend den Schritt (v) Trocknen des resultierenden Desfesoterodin in Form des Weinsäuresalzes bei reduziertem Druck, wobei die Trocknungsbedingungen so gewählt werden, dass das resultierende Desfesoterodin in Form des Weinsäuresalzes einen Restlösemittelgehalt von weniger als 2 Gew.-% aufweist.

11. Verwendung von Desfesoterodin in Form eines Weinsäuresalzes gemäß einem der Ansprüche 1 bis 7 zur Herstellung einer pharmazeutischen Formulierung, die bei oraler Einnahme im Wesentlichen bioäquivalent zu einer entsprechenden pharmazeutischen Formulierung mit Fesoterodinfumarat oder Fesoterodinhydrogenfumarat ist.

12. Verwendung einer Folie mit einer Wasserdampfdurchlässigkeit von 0,1 g/m²d bis 2,0 g/m²d zur Verpackung einer pharmazeutischen Darreichungsform enthaltend Desfesoterodin zur oralen Verabreichung, in Form eines Weinsäuresalzes gemäß einem der Ansprüche 1 bis 7.

13. Pharmazeutisches Intermediat, aufgebaut aus einem Kern (a) und einer Hülle (b), wobei
(a) der Kern Desfesoterodinweinsäuresalz gemäß einem der Ansprüche 1 bis 7 als Wirkstoff enthält und
(b) die Hülle einen oder mehrere pharmazeutische Hilfsstoffe enthält, welche die Freisetzung des Wirkstoffs modifizieren.

14. Orale Darreichungsform, bevorzugt in Form einer Tablette, enthaltend ein pharmazeutisches Intermediat gemäß Anspruch 13.

## Claims

1. (R)-2-(3-Diisopropylamino-1-phenylpropyl)-4-hydroxymethylphenol (Desfesoterodine) in the form of a tartaric acid salt, wherein the molar ratio of Desfesoterodine to tartaric acid is about 1:1.

2. Desfesoterodine salt according to claim 1, wherein the tartaric acid is L-(+)- tartaric acid.

3. Desfesoterodine salt according to claim 1 or 2 in crystalline form.

4. Desfesoterodine salt according to claim 3, wherein the crystal lattice has a layered structure.

5. Desfesoterodine salt according to claim 3 or 4, wherein the crystal system is monoclinic.

6. Desfesoterodine salt according to any one of claims 3 to 5, wherein the Desfesoterodine salt has characteristic peaks at 11.7° +/- 0.2°, 18.4 +/- 0.2°, and 18.8° +/- 0.2° (polymorph "form R") on the 2-Theta-Scale λ=1.54 A (Cu K_{α}) in a X-ray powder diffraction pattern.

7. Desfesoterodine salt according to any one of claims 3 to 6, wherein the melting point is in a range between 166 °C and 170 °C.

8. A process for the preparation of (R)-2-(3-Diisopropylamino-1-phenylpropyl)-4-hydroxymethylphenol (Desfesoterodine) in the form of a tartaric acid salt according to any one of claims 1 to 7, comprising the steps of
(i) Dissolving Desfesoterodine in a first solvent,
(ii) Dissolving tartaric acid in a second solvent,
wherein the first and the second solvent preferably have a polarity of 0.4 to 0.9 measured at 20 °C,
(iii) Combining of solutions from steps (i) and (ii),
(iv) Crystallizing of Desfesoterodine in the form of a tartaric acid salt, optionally by cooling down the solution of step (iii) to temperatures of -50 °C to 15°C; wherein steps (i) to (iii) were optionally conducted at a temperature of 20 °C to 80 °C.

9. The process according to claim 8, wherein the first and the second solvents are identical and selected from butanone and tetrahydrofuran.

10. The process according to any one of claims 8 or 9, comprising the step of (v) Drying of the resulting Desfesoterodine in the form of a tartaric acid salt at reduced pressure, wherein the drying conditions are selected in a way such that the resulting Desfesoterodine in the form of a tartaric acid salt has a remaining solvent content of less than 2% by weight.

11. Use of Desfesoterodine in the form of a tartaric acid salt according to any one of claims 1 to 7 in the manufacture of a pharmaceutical formulation, which upon oral intake essentially is a bioequivalent to a corresponding pharmaceutical formulation of Fesoterodine fumarate or Fesoterodine hydrogenfumarate.

12. Use of a film with a water vapour permeability of 0.1 g/m²d to 2.0 g/m²d for packaging a pharmaceutical dosage form for oral administration containing Desfesoterodine in the form of a tartaric acid salt according to any one claims 1 to 7.

13. Pharmaceutical intermediate, composed of a core (a) and a coating (b), wherein
(a) the core contains Desfesoterodine tartrate salt according to any one of claims 1 to 7 as the active ingredient and
(b) the coating contains one or more pharmaceutical excipients, which modify the release of the active ingredient.

14. Oral dosage form, preferably in the form of a tablet, containing a pharmaceutical intermediate according to claim 13.

## Revendications

1. (R)-2-(3-diisopropylamino-1-phénylpropyl)-4-hydroxyméthylphénol (desfésotérodine) sous forme d'un sel d'acide tartrique, dans lequel le rapport molaire de la desfésotérodine à l'acide tartrique est d'environ 1 : 1.

2. Sel de desfésotérodine selon la revendication 1, dans lequel il s'agit de l'acide L(+)-tartrique.

3. Sel de desfésotérodine selon la revendication 1 ou 2 sous forme cristalline.

4. Sel de desfésotérodine selon la revendication 3, dans lequel le réseau cristallin présente une structure à couches.

5. Sel de desfésotérodine selon la revendication 3 ou 4, dans lequel le système cristallin est monoclinique.

6. Sel de desfésotérodine selon l'une quelconque des revendications 3 à 5, le sel de desfésotérodine présentant des réflexions caractéristiques dans le diagramme de diffraction des rayons X sur poudre sur l'échelle 2-thêta À= 1,54 Å (Cu K_{α}) à 11,7° +/-0,2°, 18,4° +/- 0,2° et 18,8° +/- 0,2° (« forme R » polymorphe).

7. Sel de desfésotérodine selon l'une quelconque des revendications 3 à 6, le point de fusion se trouvant dans la plage de 166°C à 170°C.

8. Procédé de préparation de (R)-2-(3-diisopropylamino-1-phénylpropyl)-4-hydroxyméthylphénol (desfésotérodine) sous forme d'un sel d'acide tartrique selon l'une quelconque des revendications 1 à 7, comprenant les étapes consistant à :
(i) dissoudre la desfésotérodine dans un premier solvant,
(ii) dissoudre l'acide tartrique dans un deuxième solvant,
dans lequel le premier et le deuxième solvant présentent de préférence une polarité à 20°C de 0,4 à 0,9,
(iii) réunir les solutions des étapes (i) et (ii),
(iv) laisser cristalliser la desfésotérodine sous forme du sel d'acide tartrique, le cas échéant par refroidissement de la solution de l'étape (iii) à des températures de -50°C à 15°C ; les étapes (i) à (iii) étant réalisées, le cas échéant, à une température de 20 °C à 80 °C.

9. Procédé selon la revendication 8, dans lequel les premier et deuxième solvants sont identiques et sont choisis parmi la butanone ou le tétrahydrofurane.

10. Procédé selon l'une quelconque des revendications 8 ou 9, comprenant l'étape consistant à (v) sécher la desfésotérodine sous forme du sel d'acide tartrique résultante à pression réduite, dans lequel les conditions de séchage sont choisies de sorte que la desfésotérodine sous forme du sel d'acide tartrique résultante présente une teneur résiduelle en solvant inférieure à 2 % en poids.

11. Utilisation de desfésotérodine sous forme d'un sel d'acide tartrique selon l'une quelconque des revendications 1 à 7 pour la préparation d'une formulation pharmaceutique, qui, lors de la prise par voie orale, est essentiellement bioéquivalente à une formulation pharmaceutique correspondante contenant du fumarate de fésotérodine ou de l'hydrogénofumarate de fésotérodine.

12. Utilisation d'un film présentant une perméabilité à la vapeur d'eau de 0,1 g/m²j à 2,0 g/m²j pour le conditionnement d'une forme pharmaceutique destiné à l'administration par voie orale contenant de la desfésotérodine, sous la forme d'un sel d'acide tartrique selon l'une quelconque des revendications 1 à 7.

13. Intermédiaire pharmaceutique, composé d'un noyau (a) et d'une enveloppe (b), dans lequel
(a) le noyau contient du sel d'acide tartrique de desfésotérodine selon l'une quelconque des revendications 1 à 7 en tant que principe actif et
(b) l'enveloppe contient un ou plusieurs excipients pharmaceutiques, qui modifient la libération du principe actif.

14. Forme pour administration par voie orale, de préférence sous forme d'un comprimé, contenant un intermédiaire pharmaceutique selon la revendication 13.
